Europäisches Patentamt

European Patent Office

Office européen des brevets

(11) Publication number: **0 335 130**
**A1**

## (12) EUROPEAN PATENT APPLICATION

(21) Application number: 89103829.1

(22) Date of filing: 04.03.89

(51) Int. Cl.4: **B01J 23/02** , **B01J 23/06** , **B01J 23/10** , **B01J 23/12** , **C07C 2/84** , **C07C 5/32**

(30) Priority: 28.03.88 US 172808
21.11.88 US 274415
21.11.88 US 274454
21.11.88 US 274499

(43) Date of publication of application:
04.10.89 Bulletin 89/40

(84) Designated Contracting States:
DE FR GB IT NL

(71) Applicant: Institute of Gas Technology
3424 South State Street
Chicago, Illinois 60616(US)

(72) Inventor: Erekson, Erek J
22 S. Seventh Avenue
La Grange Illinois 60525(US)
Inventor: Lee, Anthony L
714 Pleasant Avenue
Glen Ellyn Illinois 60137(US)

(74) Representative: Vogel, Georg
Pat.-Ing. Georg Vogel
Hermann-Essig-Strasse 35
D-7141 Schwieberdingen(DE)

(54) Mixed basic metal oxide catalyst.

(57) A mixed basic metal oxide catalyst having the formula:
xA.yB.zC.qO
wherein A is an alkali metal selected from lithium, sodium, potassium, rubidium and cesium; B is a cation which has an ionization state 1 greater than the ionization state of C; B is selected from scandium, yttrium, lanthanum, actinium, aluminum, boron and mixtures thereof when C is selected from beryllium, magnesium, calcium, strontium, barium, radium, zinc, cadmium, mercury and mixtures thereof and B is selected from titanium, zirconium, hafnium, silicon and mixtures thereof when C is selected from scandium, yttrium, lanthanum, actinium, aluminum, boron and mixtures thereof; x and y are in mole fractions of z such that when z = 1 then x = 0.002 to 0.25, and y = 0.001 to 0.25; and q is a number necessary to maintain charge balance with O being oxygen.

The catalyst is useful for oxidative coupling of methane and aliphatic and alicyclic hydrocarbon compounds with an aromatic compound to produce higher molecular weight hydrocarbons; and for dehydrogenating hydrocarbon compounds to produce unsaturated aliphatic and alicyclic chains.

## MIXED BASIC METAL OXIDE CATALYST

## BACKGROUND OF THE INVENTION

### Field of the Invention

This invention relates to mixed basic metal oxide catalysts useful for production of higher hydrocarbons by oxidative coupling of methane, production of higher hydrocarbons by oxidative coupling of aliphatic and alicyclic hydrocarbon compounds with aliphatic and alicyclic substituted aromatic hydrocarbon compounds to form a longer substituent hydrocarbon on the aromatic ring, and production of unsaturated aliphatic and alicyclic chains by dehydrogenation of aliphatic and alicyclic hydrocarbon compounds and aliphatic and alicyclic substituted aromatic hydrocarbon compounds. Reaction of methane with oxygen in the presence of a mixed basic metal oxide catalyst in accordance with this invention results in high conversion of methane with selectivity for ethane and ethylene products. Reaction of methane with toluene and oxygen in the presence of a mixed basic metal oxide catalyst according to this invention results in high conversion to form styrene. One important dehydrogenation is the reaction of ethylbenzene in the presence of a mixed basic metal oxide catalyst according to this invention to produce styrene.

### Description of the Prior Art

Methane is currently available in large quantities from natural gas, anaerobic digestion of organic material, and chemical processing sources. However, use of methane as a chemical feedstock has been limited due to its high stability. It has been highly desirable to develop a catalyst for such reactions to enable operation under milder conditions with greater control over thermodynamic and kinetic processes as well as provide product selectivity and high reaction rate.

Oxidative coupling of methane to form higher hydrocarbons has been shown to be effected over a number of metal oxides, but yields of desired products have been low, as discussed by Keller, G.E. and M.N. Bhasin, J. of Catalysis 73, 9-19 (1982). Sodium and lead on alumina has been found to catalyze the formation of ethane and ethylene from methane, as disclosed in Hinsen, W. and M. Baerns, Chem.-Ztg., 107, 223-226 (1983) and Hinsen, W., W. Bytyn and M. Baerns, Proc. 8th Int. Congr. Catal., Berlin, III 581-592 (1984). Several U.S. patents teach a series of supported metal oxides which while effective for the conversion of methane to ethane and ethylene, are based on reducible metal oxides and used in a stoichiometric fashion by alternately exposing them to an oxidizing atmosphere and then to methane in the absence of oxygen. U.S. Patents 4,443,644; 4,443,645; 4,443,646; 4,443,647; 4,443,648; 4,443,649; 4,444,984, 4,499,322; 4,499,323; 4,499,324; and 4,523,049.

Later work has demonstrated that magnesium oxide and calcium oxide, when promoted with alkali metal salts, are active for oxidative coupling of methane to ethane and ethylene in the presence of oxygen. See Kimble, James B. and John H. Kolts, "Oxidative Coupling of Methane to Higher Hydrocarbons", Energy Progress, Vol. 6, p. 227 (1986); Driscoll, D.J., W.M. Martir, J. Wang and J.H. Lunsford, J. Am. Chem. Soc. 107, 58-63 (1985); and Ito, T., J. Wang, C. Lin and J.H. Lunsford, J. Am. Chem. Soc. 107, 5062-64 (1985). These later catalysts have the advantage of operating continuously, not requiring regeneration or pretreatment.

Borates and boron compounds have been used in partial oxidation of hydrocarbons, such as boric acid to oxidize long chain normal paraffins in the liquid phase (Illingworth, G.F. and G.W. Lester, ACS Petroleum Division Preprints, 12, No. 3, 161 (1967)) and oxidation of n-dodecane in the liquid phase to the corresponding alcohol (Lee, K.W., M.J. Choi, S.B. Kim and C.S. Choi, Ind. Eng. Chem. Res. 26, 1951 (1987)). Boric acid has been used by coating reactor walls in the combustion of methane to eliminate free radical destruction at temperatures of less than 513°C. (Kegeyan, E.M., I.S. Vardanyan and A.B. Nalbandyan, Kinetics and Catalysis 17, No. 4,749-754 and No. 4,755-759 (1976))

A number of publications describe oxidative methylation of toluene performed in Russia: Chemical Abstracts 97:127153K (1982) teaches non-catalytic methylation of toluene depended mostly on pressure

and PhMe/O/CH$_4$ molar ratio; Chemical Abstracts 99:70137t (1983) teaches oxidative methylation of toluene using a Ni-V oxide or V oxide catalyst; Chemical Abstracts l01:74734t (1984) teaches oxidative methylation of toluene in presence of 0 (max. 15 percent in reaction mixture) results in products including styrene; Chemical Abstracts 101:38205 n (1984) teaches simultaneous production of styrene, ethylbenzene, benzene, and phenols by reaction of toluene with C$_{1-4}$ alkanes in the presence of O and Fe$_2$O$_3$ or TiO$_2$ at 600-800°. Productivity increased at higher pressure in presence of H$_2$O$_2$ and/or (Me$_3$C)$_2$O$_2$; and U.S. Patent 3,830,853 teaches reaction of toluene with a lower paraffin hydrocarbon in the presence of oxygen at 600°-900° C and space velocity of 2000-10000 hour$^{-1}$.

Styrene is an important commercial unsaturated aromatic monomer used extensively in the manufacture of plastics by polymerization and copolymerization. On a commercial scale, the great majority of the world's styrene is produced by dehydrogenation of ethylbenzene A review of styrene synthesis processes is given in Kirk-Othmer, Encyclopedia of Chemical Technology, Third Edition, Vol. 21, Styrene, pgs. 770-801. One commercial process for production of styrene is the UOP Styro-Plus process using ethylbenzene and superheated steam under vacuum for the catalytic dehydrogenation of ethylbenzene as taught by Ward, D.J. et al, Hydrocarbon Processing, Vol. 66, No. 3, March 1987, pgs 47-48. Use of coke-covered alumina and boron/alumina catalysts for oxidative dehydrogenation of ethylbenzene is taught by Fiedorow, R., W. Przystajko, M. Sopa and I.G. Dalla Lana, The Nature and Catalytic Influence of Coke on Alumina: Oxidative Dehydrogenation of Ethylbenzene, Journal of Catalysis 68, pgs. 33-41 (1981). Oxidative dehydrogenation of ethylbenzene to styrene over metal pyrophosphates, such as cerium, tin, zirconium, and titanium phosphates and calcium magnesium, strontium, barium, nickel, aluminum, thorium, zinc and silicon phosphates is taught by Vrieland, G.E., Oxydehydration of Ethylbenzene to Styrene over Metal Phosphates, Journal of Catalysis 111, pgs. 1-13 (1988). This article teaches the condensed phosphate surface is the dominant factor as a catalyst and that the cation has little or no effect.

## SUMMARY OF THE INVENTION

The mixed basic metal oxide catalyst of this invention has the formula:

xA.yB.zC.qO

wherein

A is an alkali metal selected from lithium, sodium, potassium, rubidium, cesium and mixtures thereof;

B is a cation which has an ionization state 1 greater than the ionization state of C;

B is selected from scandium, yttrium, lanthanum, actinium, aluminum, boron, and mixtures thereof from Group IIIA and IIIB of the Periodic Table, preferably boron, aluminum, yttrium, and lanthanum when C is selected from beryllium, magnesium, calcium, strontium, barium, radium, zinc, cadmium, mercury and mixtures thereof from Group IIA and IIB of the Periodic Table, preferably magnesium, calcium, barium and zinc, and

B is selected from titanium, zirconium, hafnium, silicon and mixtures thereof from Group IVA and IVB of the Periodic Table, when C is selected from scandium, yttrium, lanthanum, actinium, aluminum, boron and mixtures thereof from Group IIIA and IIIB of the Periodic Table, preferably boron, aluminum, yttrium, and lanthanum;

x and y are in the mole fractions of z such that when z = 1 then x = 0.001 to 0.25, preferably 0.05 to 0.15 and y = 0.001 to 0.25, preferably 0.002 to 0.20; and

q is a number necessary to maintain charge balance with O being oxygen.

In a preferred embodiment, a boron/alkali metal promoted metal oxide catalyst having boron in amounts of about 0.2 to about 20 mole percent (about 0.05 to about 5.0 weight percent), alkali metal promoter selected from the group consisting of lithium, sodium and potassium in amounts of about 0.1 to about 25 mole percent (about 0.1 to about 40 weight percent) metal oxide selected from the group consisting of magnesium oxide, calcium oxide, zinc oxide, and barium oxide.

This invention provides a catalyst for oxidative coupling of methane to produce a higher molecular weight hydrocarbon and for oxidative coupling of aliphatic and alicyclic hydrocarbon compounds with aliphatic and alicyclic substituted aromatic hydrocarbon compounds to produce a longer substituent hydrocarbon on the aromatic ring. The reaction of an aliphatic or alicyclic hydrocarbon compound with an aliphatic or alicyclic substituted aromatic hydrocarbon compound and oxygen is conducted in the presence of a mixed basic metal oxide catalyst at elevated temperature according to the following general reaction:

$$RH + R'CH_3 + O_2 \xrightarrow{cat} R\text{-}CH_2\text{-}R' + H_2O$$

wherein R is an aliphatic or alicyclic

hydrocarbon radical; and

$R'$ is an aliphatic or alicyclic hydrocarbon radical substituted on an aromatic hydrocarbon ring.

It is unexpected that catalysts active for oxidative coupling as described above involving carbon-carbon bond formation would also be active for dehydrogenation involving carbon-hydrogen bond breaking with subsequent carbon-carbon double bond formation. Dehydrogenation of saturated organics has been described by Thomas, Charles L, Catalytic Processes and Proven Catalysts, Chap. 6, Dehydrogenation, pgs. 41-45, Academic Press (1970).

This invention provides a catalyst and process for dehydrogenation of aliphatic and alicyclic chains of aliphatic and alicyclic hydrocarbon compounds and aliphatic and alicyclic substituted aromatic hydrocarbon compounds to produce an unsaturation in the hydrocarbon chain. The reaction of an aliphatic or alicyclic hydrocarbon compound, an aliphatic or alicyclic substituted aromatic hydrocarbon compound and mixtures thereof in the dehydrogenation reaction is conducted in the presence of a mixed basic metal oxide catalyst at elevated temperature. The dehydrogenation may proceed directly according to the following general reaction of C-C bonding in a compound RH or $R'CH_3$ being converted to $C=C$ bonding $+ H_2$ or may proceed by oxidative dehydrogenation wherein C-C bonding in a compound RH or $R'CH_3 + 1/2\ O_2$ is converted to $C=C$ bonding $+ H_2O$, wherein R is an aliphatic or alicyclic hydrocarbon radical having 2 and more carbon atoms; and $R'$ is an aliphatic or alicyclic hydrocarbon radical substituted on an aromatic hydrocarbon ring. In the case of dehydrogenation of ethylbenzene to styrene according to this invention, direct dehydrogenation proceeds according to the general reaction:

$$C_6H_5C_2H_5 \xrightarrow{\text{cat}} C_6H_5C_2H_3 + H_2$$

and by partial oxidation or oxidative dehydrogenation according to the general reaction:

$$C_6H_5C_2H_5 + 1/2O_2 \xrightarrow{\text{cat}} C_6H_5C_2H_3 + H_2O.$$

## DESCRIPTION OF PREFERRED EMBODIMENTS

The catalyst of this invention is a mixed basic metal oxide catalyst having the formula xA.yB.zC.qO wherein A, B, C, x, y, z and q have the meanings set forth above with O being oxygen. The catalysts used in the process of this invention have only one oxidation state besides the metal, that is Ti, Zr, Hf and Si are only +4 and B, Al, Y and La are only +3, while Mg, Ca, Sr and Ba are only +2 and Li, K, Na, Rb and Cs are only +1. In a particularly preferred embodiment, the catalyst of this invention is a boron/alkali metal promoted metal oxide catalyst having boron in amounts of about 0.2 to about 20 mole percent (about 0.05 to about 5 weight percent) and preferably about 0.4 to about 2 mole percent (about 0.1 to about 0.5 weight percent); alkali metal promoter selected from the group consisting of lithium, sodium and potassium in amounts of about 0.1 to about 25 mole percent (about 0.1 to about 40 weight percent) and preferably about 0.5 to about 8 mole percent (about 0.5 to about 2.0 weight percent) and the remainder metal oxide selected from the group consisting of magnesium oxide, calcium oxide, zinc oxide, and barium oxide. A preferred catalyst is boron/lithium promoted magnesium oxide having about 0.20 to about 0.30 weight percent boron and about 0.8 to about 1.2 weight percent lithium.

The catalyst of this invention may be prepared by mixing water soluble ions and/or compounds of elements set forth as alkali metal (A) and cation (B) to obtain complete solution of the solids. A wide variety of non-interfering ions may be used to form suitable water soluble compounds as long as they do not cause undesired chemical interference. Suitable such compounds include acids, oxides, hydrides, and nitrates, carbonates, hydroxides, respectively. The aqueous solution of (A) and (B) are added to metal oxide (C) powder and well mixed followed by drying at a sufficient temperature and for a sufficient time to expel volatile components. The mixture is then crushed and sieved to a small size for catalytic use. Conventional and well known catalyst manufacturing techniques may be employed to produce the catalyst material noted above. When preparing these catalytic materials, it is preferred to employ manufacturing techniques resulting in a product having a substantially uniform or homogeneous composition. Shaping of the material may be effected according to conventional techniques of the art, particularly tableting, or pelleting or extrusion. The catalyst may be used unsupported or alternatively it may be supported on an inert support as known to the art, such as alumina, silica, activated carbon and the like.

The catalyst may be prepared by mixing a water soluble compound of boron, such as boric acid, boron oxides, borohydrides, and a water soluble salt of the alkali metal promoter, such as nitrate, carbonate, hydroxide or water soluble ion to obtain complete solution of the solids. The aqueous solution of boron and alkali metal is added to the metal oxide powder with stirring to obtain a homogeneous mixture which may

4

then be dried at a temperature in excess of about 110°C. The dried mixture may then be calcined at a temperature of 700° to 750°C for a sufficient time to expel volatile portions. The mixture is then crushed and sieved to an appropriately small mesh size of about -6 to about +40, preferably about -12 to about +20 for use as a catalyst.

This invention provides gas phase oxidative coupling of methane by reaction, of methane and oxygen in the presence of the above described mixed basic metal oxide catalyst, such as a boron/alkali metal promoted metal oxide catalyst. Feedstock gas comprising methane suitable for use in the process of this invention may comprise any methane containing gas which does not contain interfering compounds. Preferably, the methane containing gas used in the process of this invention comprises about 25 mole percent up to about 100 mole percent methane. Suitable sources of methane containing gas include natural gas, synthetic natural gas (SNG) product gas from gasification of carbonaceous materials, such as gasification of coal, peat, shale, and the like, as well as products of anaerobic digestion of various biomass materials. These gases principally comprise methane and may contain other hydrocarbon gases such as ethane and propane which may produce corresponding chemical reactions to those of methane in the process of this invention. Purification of such mixed gases comprising principally methane is not usually necessary. These sources of methane containing gas and processes for producing methane are well known in the art. The term "methane" as used throughout this disclosure and claims refers to methane as described above.

Any oxygen containing gas not containing interfering chemical compounds are useful as a feedstock in oxidative coupling according to this invention. The term "oxygen containing gas" as used throughout this disclosure and claims, refers to gas containing oxygen, such as air and gases having an oxygen content of up to 100 percent. It is preferred to use oxygen containing gas comprising over 50 volume percent oxygen. The mole percentage of oxygen relative to the mole percentage of methane in the gas mixture subjected to the process of this invention is about 2 to about 40 and preferably about 5 to about 20 mole percent oxygen.

The catalyst may be placed into a reactor, such as a tube-shell fixed bed, fluidized bed, moving bed, inter-bed heat exchange type, Fischer-Tropsch type, or other reactor type known to the art. Suitable reactor vessels for use at the desired operating temperatures and pressures are well known to the art. The reaction of methane and oxygen according to this invention is carried out by passing a gaseous mixture comprising methane and oxygen over the mixed basic metal oxide catalyst as defined above at about 500° to about 1100°C, preferably about 600° to about 900°C. Suitable gas residence times are about 0.002 to about 0.00002 hour preferably about 0.0005 to about 0.0001 hour. The reaction may be carried out at about pressures of about 1 to about 1515 psia, preferably about 1 to about 150 psia.

The catalyst of this invention provides a longer hydrocarbon substituent on an aromatic ring by gas phase oxidative coupling of saturated carbon atoms of an aliphatic or alicyclic hydrocarbon compound with an aliphatic or alicyclic substituted aromatic hydrocarbon and oxygen. Suitable aliphatic and alicyclic hydrocarbon compounds for use as feedstocks in the process of this invention include straight and branched chain saturated and unsaturated aliphatic hydrocarbons, such as methane, ethane, propane, butane, heptane, pentane, hexane, octane, isobutane, isohexane, isooctane, 1-pentene, 1-hexene and mixtures thereof; cyclic chain saturated and unsaturated alicyclic hydrocarbons, such as cyclobutane, cycloheptane, cycloheptene, cyclohexane, cyclohexene and mixtures thereof; and aryl substituted aliphatic and alicyclic hydrocarbons, such as toluene, xylene, mesitylene, durene, cumene and mixtures thereof. In the case of unsaturated hydrocarbons, it should be noted that the oxidative coupling of this invention does not occur at the unsaturated bonding. Suitable aliphatic and alicyclic substituted aromatic hydrocarbon compounds for use as feedstocks in this invention are aromatic ring hydrocarbons having at least one aliphatic or alicyclic hydrocarbon radical substituent on the aromatic ring, such as toluene, xylene, indan, tetralin, and mixtures thereof.

The reactions are fed to the reaction zone in mole percent proportions of about 50 to about 90 mole percent aliphatic or alicyclic hydrocarbon compounds, preferably about 75 to about 85 mole percent; about 2 to about 40 mole percent substituted aromatic hydrocarbon, preferably about 5 to about 15 mole percent; and about 2 to about 20 mole percent oxygen, preferably about 5 to about 12 mole percent. Steam may be added in an amount of up to about 1 mole of steam per mole hydrocarbon to inhibit deep oxidation. Steam does not enter into the reaction but solely acts as an oxidation inhibitor. It is preferred to use oxygen containing gas comprising over 50 volume percent oxygen. The amounts of oxygen used in the oxidative coupling of aliphatic and alicyclic hydrocarbons with aromatic hydrocarbons are expressed as pure oxygen. The oxygen containing gas may be preheated by thermal exchange with the catalyst bed to a temperature suitable for the reaction controlling step of the process. An important aliphatic feedstock suitable for use in the process of this invention may comprise methane as described above. Important substituted aromatic feedstocks include toluene and xylene available from commercial sources.

The oxidative coupling is carried out by passing the gaseous aliphatic or alicyclic hydrocarbon and aromatic feedstocks and oxygen over the mixed basic metal oxide catalyst as defined above at about 300° to about 1100°C, preferably about 600° to about 900°C. Suitable gas residence times are about 0.002 to about 0.00002 hour preferably about 0.0005 to about 0.0001 hour with space velocity of about 500 to about 50,000 vol/vol/hr, preferably about 1000 to about 5000 vol/vol/hr. The reaction may be carried out at about pressures of about 1 to about 1515 psia, preferably about 1 to about 150 psia, pressures above atmospheric may enhance the rate of reaction. Suitable reactor vessels for use at the above operating temperatures and pressures are well known to the art. The products of the single reactor used in the process of this invention may be passed to a simple separator for separation of the hydrocarbon product, condensate, and vent gas.

One important oxidative coupling reaction according to the process of this invention is the production of styrene directly by coupling of toluene and methane by the following reaction in the presence of the above defined catalyst:

$C_6H_5CH_3 + CH_4 + O_2 \rightarrow C_6H_5C_2H_3 + 2H_2O$

At 750°C the heat of reaction ($\Delta H$) is -73 kcal/mole and the sensible heat plus the heat of vaporization of toluene is about 55 kcal/mole. Thus the process operates close to autothermal conditions after initial light-off. Conventional processes using $Fe_2O_3$ as a catalyst with $Cr_2O_3$ as a stabilizer and $K_2CO_3$ as a coke retardant for production of styrene require ethylbenzene feedstock, produced from expensive benzene and ethylene and require large amounts of superheated steam (800°C and molar ratio 14 steam to 1 ethylbenzene) due to the conversion of ethylbenzene to styrene being endothermic. The process of this invention uses relatively inexpensive toluene, methane and air as feedstock to a single reactor where both styrene and ethylbenzene are produced in a process that does not require superheated steam.

The catalyst of this invention provides unsaturated aliphatic and alicyclic chains by dehydrogenation of saturated carbon atoms of an aliphatic or alicyclic hydrocarbon compound and an aliphatic or alicyclic substituted aromatic hydrocarbon and mixtures thereof. Suitable aliphatic and alicyclic hydrocarbon compounds for use as feedstocks in the process of this invention include straight and branched chain saturated aliphatic hydrocarbons, such as ethane, propane, butane, heptane, pentane, hexane, octane, isobutane, isohexane, isooctane and mixtures thereof; cyclic chain saturated alicyclic hydrocarbons, such as cyclobutane, cycloheptane, cyclohexane and mixtures thereof. Suitable aliphatic and alicyclic substituted aromatic hydrocarbon compounds for use as feedstocks in this invention are aromatic ring hydrocarbons having at least one saturated aliphatic or alicyclic hydrocarbon radical substituent on the aromatic ring, such as ethylbenzene, indan, tetralin and mixtures thereof.

The reactant is fed to the reaction zone in contact with the above defined catalyst at a space velocity of about 500 to about 50,000, preferably about 1000 to about 5000 vol/vol/hr for direct dehydrogenation and for oxidative dehydrogenation. For oxidative dehydrogenation oxygen may be added up to a mole amount of about 5 moles oxygen per mole hydrocarbon, preferably about 0.5 to about 2.0 moles oxygen per mole hydrocarbon. Steam may be added in an amount of up to about 1 mole of steam per mole hydrocarbon to inhibit undesired side reactions when oxygen is used in the feed for oxidative dehydrogenation. Steam does not enter into the reaction but solely acts as an oxidation inhibitor. For direct dehydrogenation, without oxygen in the feed, steam may be used as a heat carrying agent and up to 10 moles of steam per mole of hydrocarbon may be required.

The dehydrogenation process according to this invention is carried out by passing the gaseous aliphatic or alicyclic hydrocarbon or aromatic feedstock over the mixed basic metal oxide catalyst as defined above at a space velocity of about 500 to about 50,000 vol/vol/hr providing gas residence times of about 0.002 to about 0.00002 hour preferably about 0.0002 to about 0.00007 hour. Suitable temperatures are about 200° to about 1000°C, preferably about 600° to about 850°C for direct dehydrogenation and preferably about 450° to about 700°C for oxidative dehydrogenation. The reaction may be carried out at pressures of about 1 psia to about 1515 psia, preferably about 1 psia to about 25 psia for direct dehydrogenation and preferably about 1 psia to about 150 psia for oxidative dehydrogenation. Pressures above atmospheric may enhance the rate of reaction. Suitable reactor vessels for use at the above operating temperatures and pressures are well known to the art. The products of the single reactor used in the process of this invention may be passed to a simple separator for separation of the hydrocarbon product, condensate, and vent gas.

One important dehydrogenation reaction according to the process of this invention is the production of styrene directly by dehydrogenation of ethylbenzene or by oxidative dehydrogenation of ethylbenzene in the presence of the above defined catalyst according to the reactions set forth above. At 727°C the heat of reaction ($\Delta H$) for oxidative dehydrogenation is -29.4 kcal/mole exothermic and the sensible heat plus the heat of vaporization of ethylbenzene is about 33.0 kcal/mole. Thus the oxidative dehydrogenation process operates close to autothermal conditions after initial light-off. Conventional processes for production of styrene from ethylbenzene feedstock require large amounts of superheated steam (800°C and molar ratio 14 steam to 1 ethylbenzene) because the conversion of ethylbenzene to styrene is endothermic. The dehydration process of this invention uses a single reactor in a process that does not require superheated steam.

The specific examples are intended to be illustrative only and are not intended to limit the invention in any way.

Example I

A mixture of 3.07 grams Fisher Certified lithium nitrate and 0.43 gram Aesar 99.99 percent pure boric acid was added to a beaker. Deionized water, 50ml, was added to the beaker and stirred to obtain complete solution of the solids in the water. The aqueous solution of lithium nitrate and boric acid was slowly added to 30.0 grams alpha magnesium oxide powder and stirred to obtain a homogeneous preparation which was then dried overnight at 118°C. The composition of the mixture was 1.0 weight percent elemental lithium and 0.24 weight percent elemental boron. The mixture was then calcined for 15 minutes at 700°C and crushed and sieved to -12+20 mesh. Chemical analysis after calcining showed 0.97 weight percent elemental lithium and 0.17 weight percent elemental boron. Surface area of thee product was 2.0 meters$^2$/gram. The product was then used as a catalyst in accordance with Example X following which analysis showed 0.94 weight percent lithium, 0.20 weight percent boron, and surface area of 1.5 meters$^2$/gram.

EXAMPLES II - XV

For comparison lithium promoted magnesium oxide catalyst with 1.0 weight percent lithium (Example II) and boron promoted magnesium oxide catalyst with 0.24 weight percent boron (Example III) were prepared in a manner similar to Example I. Boron/lithium promoted magnesium oxide catalyst according to this invention was prepared having 1.0 weight percent lithium and 0.18 weight percent boron (Example IV). The promoter amounts are expressed in weight percent of the elemental lithium and boron mixed in preparation of the catalyst unless otherwise noted. Twelve grams of -12 +20 mesh of the denoted catalyst powder was supported on quartz wool in a one inch O.D. quartz tube reactor. A mixture of air and methane in mole percentages indicated were fed to the reactor maintained at the indicated temperatures and passed over the indicated catalyst where oxidative coupling of methane took place at atmospheric pressure and Weight Hourly Space Velocity (WHSV) of 5000 cm$^3$/g cat.-hr. The product gas was analyzed and conversion expressed as the percent of methane molecules that reacts; selectivity expressed as percentage of reacting methane molecules forming ethane or ethylene; and rate of ethane and ethylene formation expressed as standard cubic centimeters per gram of catalyst per hour. Results are shown in Table 1:

EP 0 335 130 A1

Table 1

| Example | Promoter Weight % | | Feed - Mole % | | Temp. | Conversion | Selectivity | Rate |
|---------|------|-------|---------|-----|------|------------|-------------|--------------|
| | Li | B | Methane | Air | °C | % | % | scc/gcat-hr. |
| II | 1.0 | - | 87 | 13 | 702 | 4.0 | 80 | 53 |
| III | - | 0.24 | 87 | 13 | 710 | 2.3 | 18 | 8 |
| IV | 1.0 | 0.18* | 87 | 13 | 700 | 4.4 | 88 | 77 |
| V | 1.0 | 1.09* | 85 | 15 | 820 | 3 | 100 | 50 |
| VI | 1.0 | 0.18 | 82 | 18 | 800 | 17 | 74 | 343 |
| VII | 1.0 | 0.18 | 80 | 20 | 801 | 19 | 66 | 354 |
| VIII | 1.0 | 0.18 | 75 | 25 | 868 | 31 | 51 | 268 |
| IX | 1.0 | - | 60 | 40 | 784 | 16.7 | 74 | 131 |
| X | - | 0.24 | 60 | 40 | 781 | 14.1 | 38 | 69 |
| XI | 1.0 | 0.12 | 60 | 40 | 799 | 16.9 | 75 | 156 |
| XII | 1.0 | 0.20 | 60 | 40 | 796 | 21.2 | 86 | 247 |
| XIII | 0.97 | 0.17* | 60 | 40 | 801 | 21.7 | 81 | 246 |
| XIV | 1.0 | 0.51 | 60 | 40 | 801 | 17.4 | 73 | 159 |
| XV | 2.3 | 0.58 | 50 | 50 | 815 | 17.0 | 73 | 204 |

* Denotes composition after calcining.

It is seen from Table 1 that the boron/lithium promoted magnesium oxide catalyst according to this invention provides greater conversion, selectivity for ethane or ethylene, and greater rate of ethane and ethylene formation per gram of catalyst than either promoter alone.


## EXAMPLE XVI

In a manner similar to Example I an aluminum/lithium promoted magnesium oxide catalyst was formed by adding 3.08 grams of Fisher Certified lithium nitrate and 2.63 grams of Fisher Certified aluminum nitrate to a beaker. Deionized water, 50 ml, was added to the beaker and stirred to obtain complete solution of the solids in the water. The aqueous solution of lithium nitrate and aluminum nitrate was slowly added to 30.0 grams of alpha magnesium oxide powder and stirred to obtain a homogeneous preparation which was then dried overnight at 118°C. The composition of the mixture was 1.0 weight percent elemental lithium and 0.6 weight percent elemental aluminum. The mixture was then calcined for 15 minutes at 700°C and crushed and sieved to -12 + 20 mesh.


## EXAMPLE XVII

The catalyst produced in accordance with Example XVI was used in the same manner as described in Examples II-XV and the results are shown in Table 2:

Table 2

| Promoter Weight % | | Feed - Mole % | | Temp. | Conversion | Selectivity | Rate |
|------|-----|---------|-----|------|------------|-------------|--------------|
| Li | Al | Methane | Air | °C | % | % | scc/gcat-hr. |
| 1.0 | 0.6 | 60 | 40 | 802 | 17.3 | 45 | 63 |

8

## EXAMPLE XVIII

A mixture of 3.08 grams Fisher Certified lithium nitrate and 4.02 grams of Alfa Yttrium nitrate Y(NO₃)-₃•6H₂O was added to a beaker. Deionized water, 50ml, was added to the beaker and stirred to obtain complete solution of the solids in the water. The aqueous solution of lithium nitrate and yttrium nitrate was slowly added to 30.0 grams alpha magnesium oxide powder and stirred to obtain a homogeneous preparation which was then dried overnight at 118°C. The composition of the mixture was 1.0 weight percent elemental lithium and 2.9 weight percent elemental yttrium. The mixture was then calcined for 15 minutes at 700°C and crushed and sieved to -12+20 mesh. Surface area of the product was 12 meters²/gram.

## EXAMPLES XIX - XX

The catalyst produced in accordance with Example XVIII was used in the same manner as described in Examples II-XV and the results are shown in Table 3:

Table 3

| Example | Promoter Weight % | | Feed - Mole % | | Temp. | Conversion | Selectivity | Rate |
|---------|------|------|---------|-----|-------|------------|-------------|--------------|
|         | Li   | Y    | Methane | Air | °C    | %          | %           | scc/gcat-hr. |
| XIX     | 1.0  | 2.9  | 60      | 40  | 799   | 20         | 68          | 158          |
| XX      | 1.0  | 2.1  | 60      | 40  | 800   | 15         | 67          | 156          |

## EXAMPLE XXI

A mixture of 3.11 grams Fisher Certified lithium nitrate and 2.11 grams Alfa lanthanum nitrate La(NO₃)-₃•6H₂O was added to a beaker. Deionized water, 50ml, was added to the beaker and stirred to obtain complete solution of the solids in the water. The aqueous solution of lithium nitrate and lanthanum nitrate was slowly added to 30.0 grams alpha magnesium oxide powder and stirred to obtain a homogeneous preparation which was then dried overnight at 118°C. The composition of the mixture was 1.0 weight percent elemental lithium and 2.3 weight percent elemental lanthanum. The mixture was then calcined for 15 minutes at 700°C and crushed and sieved to -12+20 mesh. Surface area of the product was 3.0 meters²/gram.

## EXAMPLES XXII-XXIV

The catalyst produced in accordance with Example XXI was used in the same manner as described in Examples II-XV and the results are shown in Table 4:

Table 4

| Example | Promoter Weight % | | Feed - Mole % | | Temp. | Conversion | Selectivity | Rate |
|---------|------|------|---------|-----|------|------------|-------------|-------------|
| | Li | La | Methane | Air | °C | % | % | scc/gcat-hr. |
| XXII | 1.0 | 2.3 | 60 | 40 | 805 | 18 | 74 | 180 |
| XXIII | 1.0 | 3.2 | 60 | 40 | 801 | 17 | 71 | 182 |
| XXIV | 1.0 | 4.6 | 60 | 40 | 798 | 16 | 71 | 164 |

## EXAMPLE XXV

In a manner similar to Example I a boron/lanthanum/lithium promoted magnesium oxide catalyst was formed by adding 3.07 grams of Fisher Certified lithium nitrate, 3.03 grams of Alpha lanthanum nitrate hexahydrate and 0.57 gram of boric acid (Aesar 99.99 percent pure) to a beaker. Deionized water, 50ml, was added to the beaker and stirred to obtain complete solution of the solids in the water. The aqueous solution of lithium nitrate, lanthanum nitrate and boric acid was slowly added to 30.0 grams of Alpha (-325 mesh) magnesium oxide powder and stirred to obtain a homogeneous preparation which was then dried overnight at 115°C. The composition of the mixture was 1.0 weight percent elemental lithium, 3.2 weight percent elemental lantanum and 0.35 weight percent elemental boron. The mixture was then calcined for 15 minutes at 700°C and crushed and sieved to -12 + 20 mesh.

## EXAMPLE XXVI

Gaseous feedstock was fed to a tubular reactor containing a packed bed of 0.06Li:0.009La:0.012B:1.0MgO catalyst prepared as set forth in Example XXV. The feedstock comprised methane/air/toluene in proportions of 60/40/5 mole fraction. A single pass was made at 3000 SCF/h-ft³, atmospheric pressure, and 665°C resulting in a product isolated in an ice trap and analyzed as follows:

| | Weight percent |
|---|---|
| Toluene | 78.3 |
| Benzene | 1.4 |
| Ethyl benzene | 8.5 |
| Styrene | 4.4 |
| Xylenes | 0.9 |
| Propyl benzene | 1.0 |
| Diphenyl ethane | 1.7 |
| Other aromatics | 3.8 |

## Claims

1. A mixed basic metal oxide catalyst having the formula:

xA.yB.zC.qO

wherein

A is an alkali metal selected from lithium, sodium, potassium, rubidium and cesium;

B is a cation which has an ionization state 1 greater than the ionization state of C;

B is selected from scandium, yttrium, lanthanum, actinium, aluminum, boron and mixtures thereof when C is selected from beryllium, magnesium, calcium, strontium, barium, radium, zinc, cadmium, mercury and mixtures thereof and

B is selected from titanium, zirconium, hafnium, silicon and mixtures thereof when C is selected from scandium, yttrium, lanthanum, actinium, aluminum, boron and mixtures thereof;

x and y are in mole fractions of z such that when $z = 1$ then $x = 0.002$ to $0.25$, and $y = 0.001$ to $0.25$; and

q is a number necessary to maintain charge balance with O being oxygen.

2. A catalyst according to Claim 1 wherein B is selected from the group consisting of boron, aluminum, yttrium and lanthanum and C is selected from the group consisting of magnesium, calcium, barium and zinc.

3. A catalyst according to Claim 1 wherein B is selected from the group consisting of silicon, titanium, zirconium and hafnium and C is selected from the group consisting of boron, aluminum, yttrium and lanthanum.

4. A catalyst according to Claim 1 wherein $x = 0.05$ to $0.15$ and $y = 0.002$ to $0.20$.

5. A catalyst according to Claim 1 wherein said A is present in about 0.5 to about 8 mole percent and said B is present in about 0.4 to about 2 mole percent.

6. A catalyst according to Claim 1 wherein A is lithium.

7. A catalyst according to Claim 1 wherein C is magnesium.

8. A catalyst according to Claim 1 wherein A is lithium and C is magnesium.

9. A catalyst according to Claim 1 wherein B is boron and is present in about 0.2 to about 20 mole percent.

10. A catalyst according to Claim 9 wherein A is lithium.

11. A process for producing higher molecular weight hydrocarbons from a gas comprising methane, said process comprising:

oxidative coupling said methane with oxygen in the presence of a mixed basic metal oxide catalyst having the formula:

$xA.yB.zC.qO$

wherein

A is an alkali metal selected from lithium, sodium, potassium, rubidium, cesium and mixtures thereof;

B is a cation which has an ionization state 1 greater than the ionization state of C;

B is selected from the group consisting of scandium, yttrium, lanthanum, actinium, aluminum, boron and mixtures when C is selected from the group consisting of beryllium, magnesium, calcium, strontium, barium, radium, zinc, cadmium, mercury and mixtures thereof, and

B is selected from the group consisting of titanium, zirconium, hafnium, silicon and mixtures thereof, when C is selected from the group consisting of scandium, yttrium, lanthanum, actinium, aluminum, boron and mixtures thereof;

x and y are in mole fractions of z such that when $z = 1$ then $x = 0.001$ to $0.25$,

and $y = 0.001$ to $0.25$; and

q is a number necessary to maintain charge balance with O being oxygen.

12. A process for producing higher molecular weight hydrocarbons by forming longer substituent hydrocarbon on an aromatic ring, said process comprising:

oxidative coupling a compound selected from aliphatic hydrocarbon compounds, alicyclic hydrocarbon compounds, and mixtures thereof with a compound selected from aliphatic substituted aromatic hydrocarbon compounds, alicyclic substituted aromatic hydrocarbon compounds, and mixtures thereof in the presence of oxygen and a mixed basic metal oxide catalyst having the formula:

$xA.yB.zC.qO$

wherein

A is an alkali metal selected from lithium, sodium, potassium, rubidium, cesium and mixtures thereof;

B is a cation which has an ionization state 1 greater than the ionization state of C;

B is selected from the group consisting of scandium, yttrium, lanthanum, actinium, aluminum, boron and mixtures when C is selected from the group consisting of beryllium, magnesium, calcium, strontium, barium, radium, zinc, cadmium, mercury and mixtures thereof, and

B is selected from the group consisting of titanium, zirconium, hafnium, silicon and mixtures thereof, when C is selected from the group consisting of scandium, yttrium, lanthanum, actinium, aluminum, boron and mixtures thereof;

x and y are in mole fractions of z such that when $z = 1$ then $x = 0.001$ to $0.25$, and $y = 0.001$ to $0.25$; and

q is a number necessary to maintain charge balance with O being oxygen.

13. A process according to Claim 12 wherein said aliphatic hydrocarbon compound is methane and said aromatic hydrocarbon compound is toluene.

14. A process for producing unsaturated aliphatic and alicyclic hydrocarbon chains by dehydrogenation, said process comprising:

dehydrogenating a compound selected from aliphatic hydrocarbon compounds, alicyclic hydrocarbon compounds, aliphatic substituted aromatic hydrocarbon compounds, alicyclic substituted aromatic hydrocarbon compounds, and mixtures thereof in the presence of a mixed basic metal oxide catalyst having the formula:

$xA.yB.zC.qO$

wherein A is an alkali metal selected from lithium, sodium, potassium, rubidium, cesium and mixtures thereof;

B is a cation which has an ionization state 1 greater than the ionization state of C;

B is selected from the group consisting of scandium, yttrium, lanthanum, actinium, aluminum, boron and mixtures thereof when C is selected from the group consisting of beryllium, magnesium, calcium, strontium, barium, radium, zinc, cadmium, mercury and mixtures thereof, and

B is selected from the group consisting of titanium, zirconium, hafnium, silicon and mixtures thereof, when C is selected from the group consisting of scandium, yttrium, lanthanum, actinium, aluminum, boron and mixtures thereof;

x and y are in mole fractions of z such that when $z = 1$ then x-0.001 to 0.25, and $y = 0.001$ to 0.25; and

q is a number necessary to maintain charge balance with O being oxygen.

15. A process according to Claim 14 wherein said hydrocarbon compound is ethylbenzene.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 183 225 (NIPPON SHOKUBAI K.K.) * Abstract; pages 6-8; examples 16,19,33 * | 1,2,4-10 | B 01 J 23/02 |
| | --- | | B 01 J 23/06 |
| A | EP-A-0 177 327 (STANDARD OIL) | | B 01 J 23/10 |
| | --- | | B 01 J 23/12 |
| A | EP-A-0 112 754 (IFP) | | C 07 C 2/84 |
| | --- | | C 07 C 5/32 |
| A | FR-A-2 104 462 (GOODYEAR) | | |
| | --- | | |
| A | US-A-3 849 292 (W.K.T. GLEIM) | | |
| | --- | | |
| A | US-A-4 656 155 (JOSEFOWICZ) | | |
| | --- | | |
| D,A | US-A-4 499 324 (GAFFNEY) | | |
| | ----- | | |

TECHNICAL FIELDS SEARCHED (Int. Cl.4)

B 01 J
C 07 C

The present search report has been drawn up for all claims

| Place of search | Date of completion of the search | Examiner |
|---|---|---|
| THE HAGUE | 24-05-1989 | LO CONTE C. |

EPO FORM 1503 03.82 (P0401)